(19) Europäisches Patentamt
European Patent Office
Office européen des brevets

(11) Publication number: 0 122 130 B1

(12) # EUROPEAN PATENT SPECIFICATION

(45) Date of publication of patent specification: 06.09.89

(51) Int. Cl.$^4$: C 07 D 417/00, A 01 N 43/78

(21) Application number: 84302362.3

(22) Date of filing: 06.04.84

(54) **Herbicidal tetrahydrobenzothiazole derivatives.**

(30) Priority: 08.04.83 JP 61927/83

(43) Date of publication of application: 17.10.84 Bulletin 84/42

(45) Publication of the grant of the patent: 06.09.89 Bulletin 89/36

(84) Designated Contracting States: DE FR GB IT

(56) References cited:
US-A-3 379 725

(73) Proprietor: KUREHA KAGAKU KOGYO KABUSHIKI KAISHA
9-11 Horidome-cho 1-chome Nihonbashi Chuo-ku
Tokyo 103 (JP)

(72) Inventor: Aoki, Katsumichi
16-10 Nishi-Arata Ueda-machi
Iwaki-shi Fukushima-ken (JP)
Inventor: Shida, Takafumi
1-6 Ochiai Nishiki-machi
Iwaki-shi Fukushima-ken (JP)
Inventor: Kumazawa, Satoru
16-1 Maehara Nishiki-machi
Iwaki-shi Fukushima-ken (JP)
Inventor: Shimizu, Susumu
62 Sagiuchi Nishiki-machi
Iwaki-shi Fukushima-ken (JP)
Inventor: Kanda, Yoichi
45-2 Ohshima Nishiki-machi
Iwaki-shi Fukushima-ken (JP)
Inventor: Satake, Keigo
4-8-15 Nakaoka-machi Iwaki-shi
Fukushima-ken (JP)
Inventor: Yamazaki, Shiro
11-1 Kaneyama Joban-Nishigomachi
Iwaki-shi Fukushima-ken (JP)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 122 130 B1

Courier Press, Leamington Spa, England.

(72) Inventor: **Shinkawa, Hiroyasu**
**14 Suka, Nishiki-machi**
**Iwaki-shi Fukushima-ken (JP)**
Inventor: **Chida, Tsuneaki**
**16-1 Maehara Nishiki-machi**
**Iwaki-shi Fukushima-ken (JP)**
Inventor: **Arabori, Hideo**
**16-1 Maehara Nishiki-machi**
**Iwaki-shi Fukushima-ken (JP)**
Inventor: **Watanabe, Takeo**
**78-31 Hananoi Nishiki-machi**
**Iwaki-shi Fukushima-ken (JP)**

(74) Representative: **Myerscough, Philip Boyd et al**
**J.A.Kemp & Co. 14, South Square Gray's Inn**
**London, WC1R 5EU (GB)**

**Description**

The present invention relates to derivatives of tetrahydrobenzothiazole, their preparation, herbicidal compositions containing them and a method of controlling the growth of weeds using them.

The present inventors have sought to find a compound showing excellent activity in selectively controlling weeds such *Echinochloa crus-galli, Poa annua, Portulaca oleracea, Cardamine flexuosa,* without being phytotoxic to crop plants such as rice, wheat, soybean and maize. As a result, they have found that derivatives of tetrahydrobenzothiazole represented by the formula (I) below show an excellent herbicidal activity for controlling the weeds in practice.

US—A—3,379,725 discloses herbicidal compounds of formula:

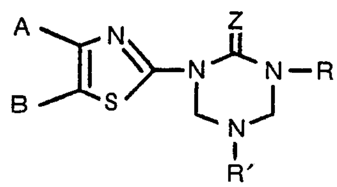

wherein A and B may together form a hydrocarbon bridge having 3 to 5 carbon atoms, for example, a 1,4-butadiene bridge, Z is an oxygen or sulphur atom, R is a hydrocarbon radical containing 1 to 4 carbon atoms and R′ is a hydrocarbon radical containing 1 to 6 carbon atoms, which may optionally contain heteroatoms.

The compounds represented by formula (I) are novel, and of course, their physiological properties have never been known. According to herbicidal tests consisting essentially of foliar application and soil treatment, the derivatives of tetrahydrobenzothiazole according to the present invention (hereinafter referred to as "the present compounds") show excellent herbicidal activity on broad-leaved weeds, for instance *Stellaria media, Cardamine flexuosa* and *Portulaca oleracea,* Cyperaceous weeds, for instance *Cyperus iria*, and Gramineous weeds, for instance those belonging to the genus *Echinochloa* and *Poa annua,* and particularly show strong herbicidal activity when applied to the leaves and stems of these weeds. The present compounds may be applied to crop areas such as paddy fields, upland fields and orchards and also to non-crop areas.

According to the present invention, there is provided a derivative of tetrahydrobenzothiazole represented by the formula (I):

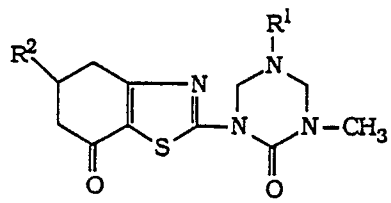

(I)

wherein $R^1$ and $R^2$ represent independently a straight-chain alkyl group having from 1 to 6 carbon atoms or a branched-chain alkyl group having from 3 to 6 carbon atoms.

The present invention further provides a herbicidal composition comprising as active ingredient at least one derivative of tetrahydrobenzothiazole represented by the formula (I), together with a carrier or diluent therefor.

The present invention also provides a method of controlling the growth of weeds at a locus, which method comprises applying to the locus a herbicidally effective amount of a derivative of tetrahydrobenzothiazole of formula (I).

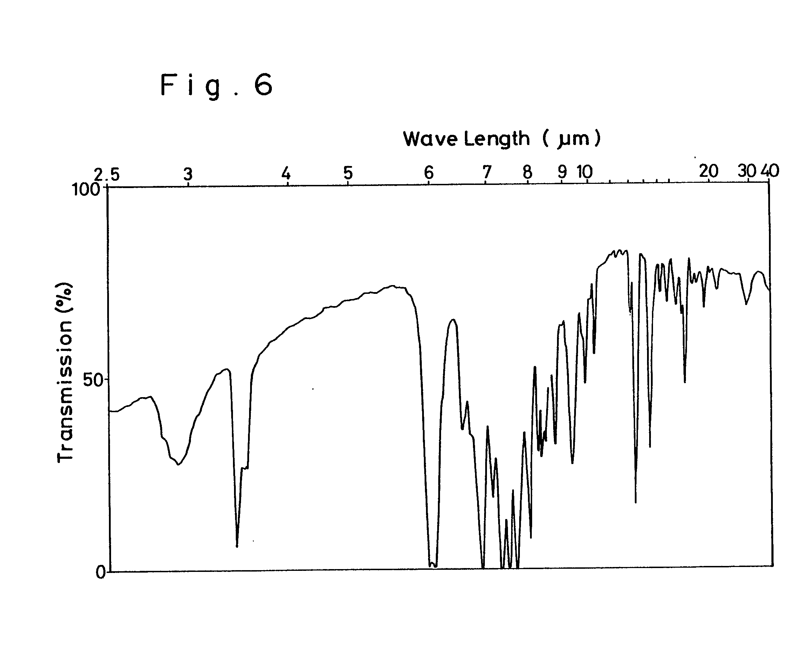

In the attached Drawings, Figs. 1 to 6 show the infra-red absorption spectra of Compounds Nos. 1 to 6 according to the present invention, respectively.

The compounds represented by the formula (I) are synthesized by reacting a compound of formula (II):

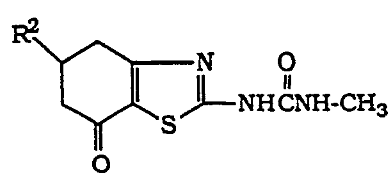

(II)

wherein $R^2$ is as defined above, with formaldehyde and an amine of formula $R^1—NH_2$ wherein $R^1$ is as defined above. One mole of the compound of formula (II) reacts with two moles of formaldehyde and one mole of the amine of formula $R^1—NH_2$.

Compounds of the present invention are shown in Table 1 together with their respective physical properties:

Table 1

| Number of the present compound | Substituents in the formula (I) $R^1$ | $R^2$ | Melting point (°C) | Yield (%) | Infrared spectrum |
|---|---|---|---|---|---|
| 1 | $CH_3$- | $CH_3$- | 175 to 176 | 58 | Fig. 1 |
| 2 | $CH_3CH_2CH_2$- | $CH_3$- | 136 to 138 | 93 | Fig. 2 |
| 3 | $CH_3$- | $(CH_3)_2CH$- | 170 to 172 | 73 | Fig. 3 |
| 4 | $CH_3CH_2CH_2$- | $(CH_3)_2CH$- | 121 to 123 | 68 | Fig. 4 |
| 5 | $CH_3$- | $(CH_3)_3C$- | 186 to 187 | 95 | Fig. 5 |
| 6 | $(CH_3)_2CH$- | $(CH_3)_3C$- | 163 to 164 | 63 | Fig. 6 |

The present invention will be explained in more detail in the following Examples.
The NMR spectra of the compounds are measured using TMS as the internal standard.
The following symbols are used:
S means singlet, d means doublet, t means triplet, 6-plet means sextet and m means multiplet.

Example 1

Synthesis of Tetrahydro-1-methyl-3-(4,5,6,7-tetrahydro-5-methyl-7-oxo-2-benzothiazolyl)-5-propyl-1,3,5-triazine-2(1H)-one (Compound No. 2)

To a solution of 1.5 g (0.0063 mol) of N-methyl-N'-(4,5,6,7-tetrahydro-5-methyl-7-oxo-2-benzothiazolyl) urea in 20 ml of dimethylformamide, 1.8 ml of aqueous 35% solution of formaldehyde (0.021 mol) were added, and the mixture was stirred for 30 min at room temperature. After adding 0.37 g (0.0063 mol) of propylamine to the mixture, the whole mixture was stirred for 18 hours at room temperature and for a further 3 hours at 90°C.

The solid material obtained by distilling off the solvents and volatile substances from the mixture was recrystallized from ethanol to obtain 1.55 g of yellow crystals (yield: 93% and M.P.: 138°C) showing the following spectra.

IR (as KBr tablet) $cm^{-1}$: $\nu_{CO}$ 1670 and 1640.

NMR (in $CDCl_3$)δ ppm: 0.97 (3H, t, J=7Hz, 5-$CH_2CH_2CH_3$); 1.22 (3H, bs, 5'-$CH_3$); 1.58 (2H, 6-plet, J=7Hz, 5-$CH_2CH_2CH_3$); 2.10 to 2.58 (5H, 4'-$H_2$, 6'-$H_2$ and 5'-H); 2.73 (2H, t, J=7Hz, 5-$CH_2CH_2CH_3$); 3.02 (3H, s, 1-$CH_3$); 4.32 (2H, s, 6-$H_2$) and 5.15 (2H, s, 4-$H_2$).

Example 2

Synthesis of Tetrahydro-1,5-dimethyl-3-(4,5,6,7-tetrahydro-7-oxo-5-isopropyl-2-benzothiazolyl)-1,3,5-triazine-2(1H)-one (Compound No. 3)

To a solution of 3.4 g (0.013 mol) of N-methyl-N'-(4,5,6,7-tetrahydro-7-oxo-5-isopropyl-2-benzothiazolyl) urea in 20 ml of dimethylformamide, 3.6 ml of aqueous 35% solution of formaldehyde (0.042 mol) were added, and after stirring the mixture for 30 min at room temperature, 1.9 ml of aqueous 40% solution of methylamine (0.025 mol) was added to the mixture. The whole mixture was stirred for 30 min at room temperature and for a further one hour at 70 to 80°C. Then, the reaction mixture was poured into water, and the thus obtained crystalline precipitate was collected by filtration and recrystallized from ethanol to obtain 2.9 g of pale yellow crystals (yield: 73% and M.P.: 170 to 172°C).

The product showed the following spectra.

IR (as KBr tablet $cm^{-1}$): $\nu_{CO}$ 1640.

NMR (in $CDCl_3$ δ ppm: 1.00 (6H, d, J=6Hz,

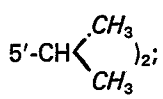

1.36 to 3.30 (6H, m, 4'-$H_2$, 6'-$H_2$, 5'-H and 5'-*CH*$(CH_3)_2$); 2.70 (3H, s, 5-$CH_3$); 3.07 (3H, s, 1-$CH_3$); 4.39 (2H, s, 6-$H_2$) and 5.22 (2H, s, 4-$H_2$).

The following are Preparation Examples wherein the carrier (diluent), the adjuvant, the mixing proportions thereof and the active ingredient may be changed.

Preparation Example 1

Preparation and Use of a Wettable Powder

By mixing 50 parts by weight of the present compound (Compound No. 1), 5 parts by weight of a salt of ligninsulfonic acid, 3 parts by weight of a salt of alkylsulfonic acid and 42 parts by weight of diatomaceous earth, and pulverizing the mixture, a wettable powder was prepared.

The thus prepared wettable powder is applied after diluting thereof with water to a suitable concentration of the present compound (Compound No. 1) as the active ingredient.

Preparation Example 2

Preparation and Application of an Emulsifiable Concentrate

By uniformly mixing 25 parts by weight of the present compound (Compound No. 3), 65 parts by weight of xylene and 10 parts by weight of polyoxyethylenealkyl aryl ether, an emulsifiable concentrate was prepared. The thus prepared emulsifiable concentrate is applied after diluting thereof with water to a suitable concentration of the present compound (Compound No. 3) as the active ingredient.

Preparation Example 3

Preparation and Application of a Granular Composition

After uniformly mixing 8 parts by weight of the present compound (Compound No. 5), 40 parts by weight of bentonite, 45 parts by weight of clay and 7 parts by weight of a salt of lignin-sulfonic acid, the mixture was kneaded with water and processed into granules by an extruding granulator. The granules were dried and sifted to provide a granular composition which is directly applied.

The effectiveness of the present compounds is shown below.

Herbicidal Test Example 1

To the foliage of each of the following plants grown from their seeds under a management in a plastic planter of 180 × 580 × 150 mm in size, each of the wettable powders prepared as in Preparation Example 1 and diluted to 0.1 part by weight of the active ingredient with water was sprayed by a small pressured-sprayer at a rate of 10 litres per are (100 $m^2$). After spraying, the plastic planters were placed in a greenhouse.

After 21 days of the treatment, the state of the plants in the planter was observed to assess the damage due to the application of each of the wettable powders to find out the herbicidal activity thereof according to the following criteria.

Criteria of Herbicidal Activity

| Index | Phytotoxicity |
|---|---|
| 0 | none |
| 1 | very slight |
| 2 | slight |
| 3 | moderate |
| 4 | severe |
| 5 | very severe (plants withered) |

Name of the plants tested

| | |
|---|---|
| 1. *Echinochloa crus-galli* | 2. *Digitaria ciliaris* |
| 3. *Poa annua* | 4. *Cyperus iria* |
| 5. *Chenopodium album* | 6. *Stellaria media* |
| 7. *Cardamine flexuosa* | 8. *Portulaca oleracea* |
| 9. *Glycine max* (Soybean) | 10. *Zea mays* (maize) |
| 11. *Triticum aestivum* (wheat) | |

The herbicidal activities of the present compounds thus assessed are shown in Table 2. The growth state of the plants when the present wettable powders were applied was the 2 to 4-leaf-stage.

Table 2: Herbicidal Activity

| Plant \ Present Compound No. | 1 | 2 | 3 | 4 | 5 | 6 | Not treated |
|---|---|---|---|---|---|---|---|
| *Echinochloa crus-galli* | 3 | 4 | 3 | 3 | 4 | 4 | 0 |
| *Digitaria ciliaris* | 5 | 5 | 4 | 4 | 5 | 5 | 0 |
| *Poa annua* | 5 | 5 | 3 | 5 | 5 | 5 | 0 |
| *Cyperus iria* | 2 | 2 | 1 | 1 | 3 | 3 | 0 |
| *Chenopodium album* | 5 | 5 | 3 | 3 | 5 | 5 | 0 |
| *Stellaria media* | 5 | 5 | 5 | 5 | 5 | 5 | 0 |
| *Cardamine flexuosa* | 5 | 5 | 5 | 5 | 5 | 5 | 0 |
| *Portulaca oleracea* | 5 | 4 | 3 | 2 | 5 | 5 | 0 |
| *Glycine max* (soy-bean) | 1 | 0 | 0 | 0 | 1 | 1 | 0 |
| *Zea mays* (maize) | 0 | 0 | 0 | 0 | 0 | 0 | 0 |
| *Triticum aestivum* (wheat) | 1 | 0 | 1 | 0 | 0 | 0 | 0 |

## Claims

1. A derivative of tetrahydrobenzothiazole represented by the formula (I):

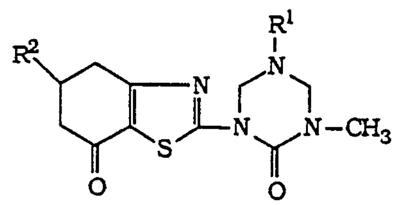

(I)

wherein $R^1$ and $R^2$ represent independently a straight-chain alkyl group having from 1 to 6 carbon atoms or a branched-chain alkyl group having from 3 to 6 carbon atoms.

2. A compound according to claim 1 in which $R^1$ is methyl, n-propyl or isopropyl.

3. A compound according to claim 1 or 2 in which $R^2$ is methyl, isopropyl or tert.butyl.

4. A compound according to claim 1 which is tetrahydro-1-methyl-3-(4,5,6,7-tetrahydro-5-methyl-7-oxo-2-benzothiazolyl)-5-propyl-1,3,5-triazine-2(1H)-one.

5. A compound according to claim 1 which is tetrahydro-1,5-dimethyl-3-(4,5,6,7-tetrahydro-7-oxo-5-isopropyl-2-benzothiazolyl)-1,3,5-triazine-2(1H)-one.

6. A compound according to claim 1 which is tetrahydro-1-methyl-3-(4,5,6,7-tetrahydro-5-t-butyl-7-oxo-2-benzothiazolyl)-5-methyl-1,3,5-triazine-2(1H)-one.

7. A compound according to claim 1 which is tetrahydro-1-methyl-3-(4,5,6,7-tetrahydro-5-t-butyl-7-oxo-2-benzothiazolyl)-5-isopropyl-1,3,5-triazine-2(1H)-one.

8. A process for the preparation of a derivative of tetrahydrobenzothiazole of formula (I) as defined in claim 1, which process comprises reacting a compound of formula (II):

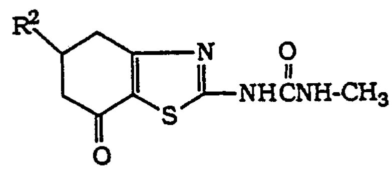

(II)

wherein $R^2$ is as defined in claim 1, with formaldehyde and an amine of formula $R^1—NH_2$ wherein $R^1$ is as defined in claim 1.

9. A herbicidal composition comprising as active ingredient at least one derivative of tetrahydrobenzothiazole represented by the formula (I) as defined in claim 1, together with a carrier or diluent therefor.

10. A method of controlling the growth of weeds at a locus, which method comprises applying to the locus a herbicidally effective amount of a derivative of tetrahydrobenzothiazole of formula (I) as defined in claim 1.

## Patentansprüche

1. Tetrahydrobenzothiazolderivat der Formel (I)

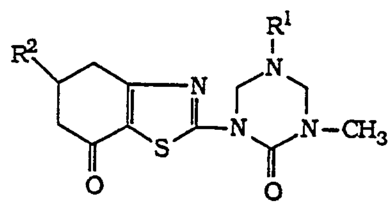

(I)

worin $R^1$ und $R^2$ unabhängig voneinander geradkettige Alkylgruppen mit 1 bis 6 Kohlenstoffatomen oder verzweigtkettige Alkylgruppen mit 3 bis 6 Kohlenstoffatomen bedeuten.

2. Verbindung nach Anspruch 1, worin $R^1$ Methyl, n-Propyl oder Isopropyl bedeutet.

3. Verbindung nach Anspruch 1 oder 2, worin $R^2$ Methyl, Isopropyl oder tert.-Butyl bedeutet.

4. Verbindung nach Anspruch 1, nämlich Tetrahydro-1-methyl-3-(4,5,6,7-tetrahydro-5-methyl-7-oxo-2-benzothiazolyl)-5-propyl-1,3,5-triazin-2(1H)-on.

5. Verbindung nach Anspruch 1, nämlich Tetrahydro-1,5-dimethyl-3-(4,5,6,7-tetrahydro-7-oxo-5-isopropyl-2-benzothiazolyl)-1,3,5-triazin-2(1H)-on.

6. Verbindung nach Anspruch 1, nämlich Tetrahydro-1-methyl-3-(4,5,6,7-tetrahydro-5-tert.-butyl-7-oxo-2-benzothiazolyl)-5-methyl-1,3,5-triazin-2(1H)-on.

7. Verbindung nach Anspruch 1, nämlich Tetrahydro-1-methyl-3-(4,5,6,7-tetrahydro-5-tert.-butyl-7-oxo-2-benzothiazolyl)-5-isopropyl-1,3,5-triazin-2(1H)-on.

8. Verfahren zur Herstellung des Tetrahydrobenzothiazolderivats der Formel (I) gemäß Anspruch 1, welches Verfahren darin besteht, eine Verbindung der Formel (II)

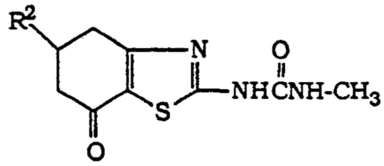

(II)

worin $R^2$ die in Anspruch 1 angegebenen Bedeutungen besitzt, mit Formaldehyd und einem Amin der Formel $R^1$—$NH_2$, worin $R^1$ die in Anspruch 1 angegebenen Bedeutungen besitzt, umzusetzen.

9. Herbizide Zubereitung enthaltend als Wirkstoff mindestens ein Tetrahydrobenzothiazolderivat der in Anspruch 1 definierten Formel (I) zusammen mit einem Träger der Verdünnungsmittel dafür.

10. Verfahren zur Beeinflussung des Wachstums von Unkräutern in ihrem Wachstumsbereich, welches Verfahren darin besteht, eine herbizid wirksame Menge eines Tetrahydrobenzothiazolderivats der in Anspruch 1 definierten Formel (I) auf den Bereich aufzubringen.

**Revendications**

1. Dérivés du tétrahydrobenzothiazole représenté par la formule (I):

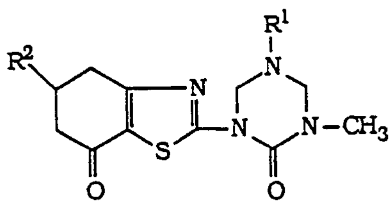

(I)

dans laquelle $R^1$ et $R^2$ représentent indépendamment un groupe alkyle linéaire en $C_1$ à $C_6$ ou un groupe alkyle ramifié en $C_3$ à $C_6$.

2. Composé suivant la revendication 1, dans lequel $R^1$ est un groupe méthyle, n-propyle ou isopropyle.

3. Composé suivant la revendication 1 ou 2, dans lequel $R^2$ est un groupe méthyle, isopropyle ou tert-butyle.

4. Composé suivant la revendication 1, qui est la tétrahydro-1-méthyl-3-(4,5,6,7-tétrahydro-5-méthyl-7-oxo-2-benzothiazolyl)-5-propyl-1,3,5-triazine-2(1H)-one.

5. Composé selon la revendication 1, qui est la tétrahydro-1,5-diméthyl-3-(4,5,6,7-tétrahydro-7-oxo-5-iso-propyl-2-benzothiazolyl)-1,3,5-triazine-2(1H)-one.

6. Composé selon la revendication 1, qui est la tétrahydro-1-méthyl-3-(4,5,6,7-tétrahydro-5-t-butyl-7-oxo-2-benzothiazolyl)-5-méthyl-1,3,5-triazine-2(1H)-one.

7. Composé suivant la revendication 1, qui est la tétrahydro-1-méthyl-3-(4,5,6,7-tétrahydro-5-t-butyl-7-oxo-2-benzothiazolyl)-5-isopropyl-1,3,5-triazine-2(1H)-one.

8. Procédé de préparation d'un dérivé du tétrahydrobenzothiazole répondant à la formule (I) tel que défini dans la revendication 1, lequel procédé comprend le fait de faire réagir un composé répondant à la formule (II):

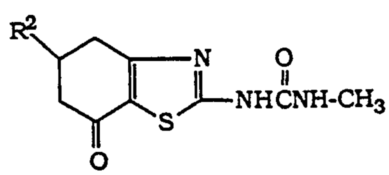

(II)

dans laquelle $R^2$ est tel que défini dans la revendication 1, avec du formaldéhyde et une amine répondant à la formule $R^1$—$NH_2$, dans laquelle $R^1$ est tel que défini dans la revendication 1.

9. Composition herbicide comprenant comme ingrédient actif au moins un dérivé du tétrahydrobenzothiazole représenté par la formule (I) tel que défini dans la revendication 1, en même temps qu'un support ou diluant pour celui-ci.

10. Procédé de lutte contre le développement des mauvaises herbes sur leur emplacement, lequel procédé comprend l'application sur l'emplacement d'une quantité efficace comme herbicide d'un dérivé de tétrahydrobenzothiazole répondant à la formule (I) tel que défini dans la revendication 1.

Fig. 1

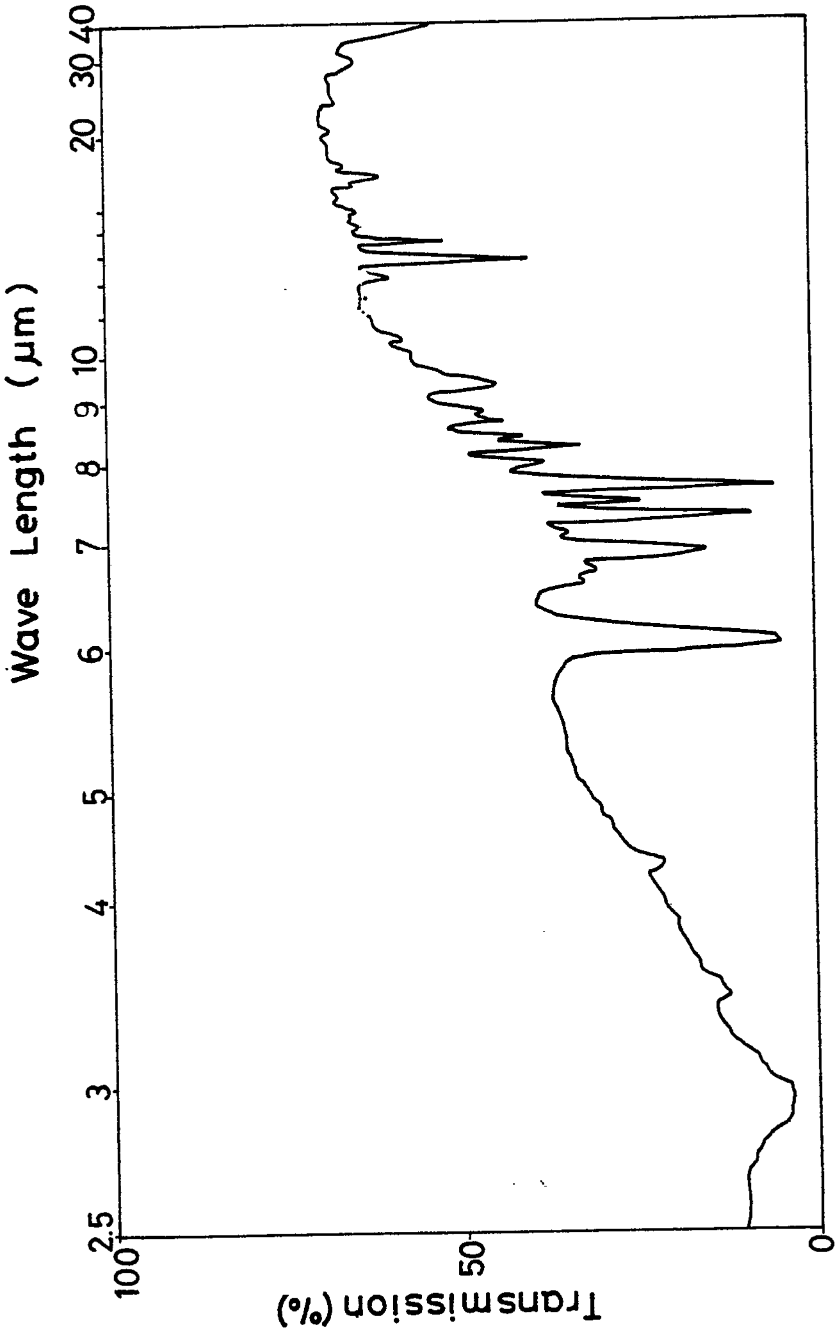

Fig. 2

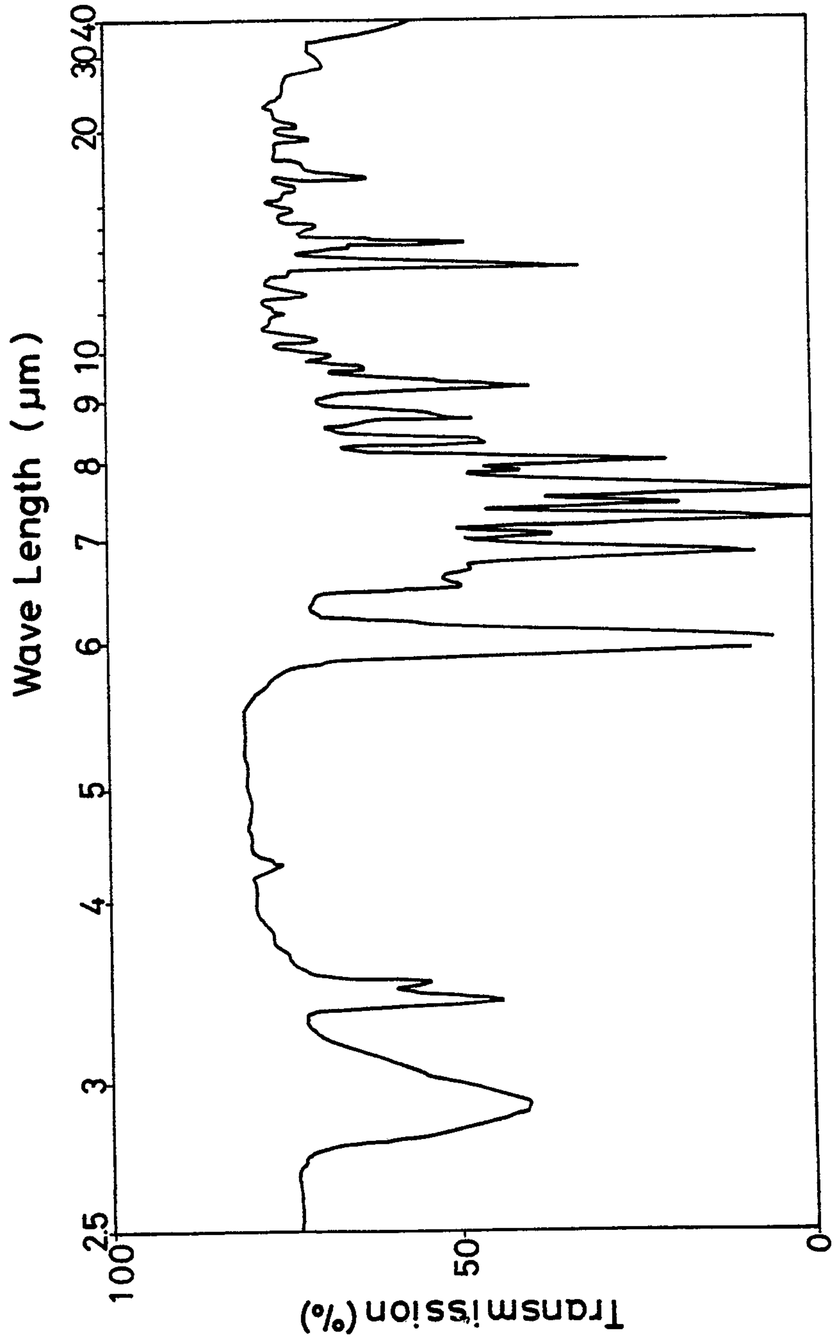

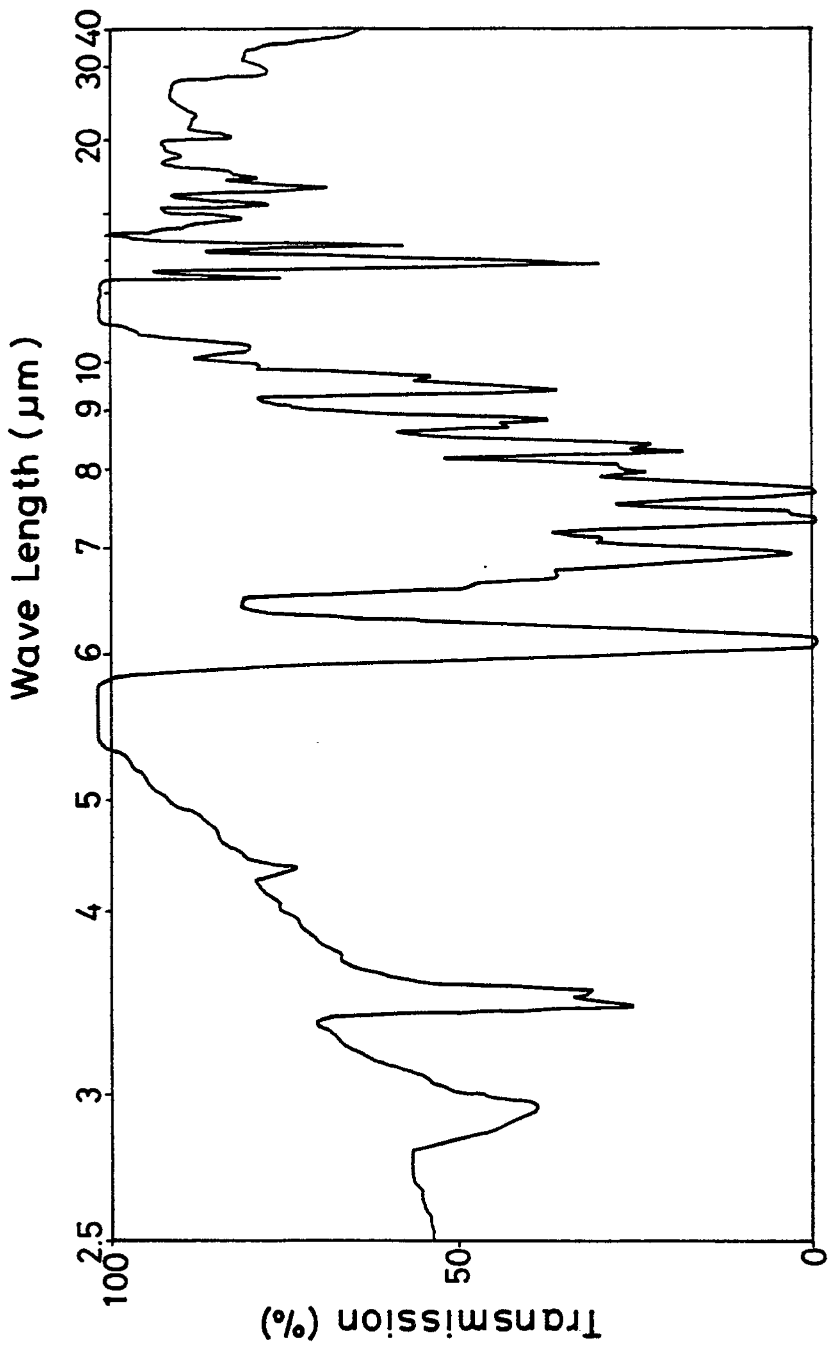


Fig. 3

Fig. 4

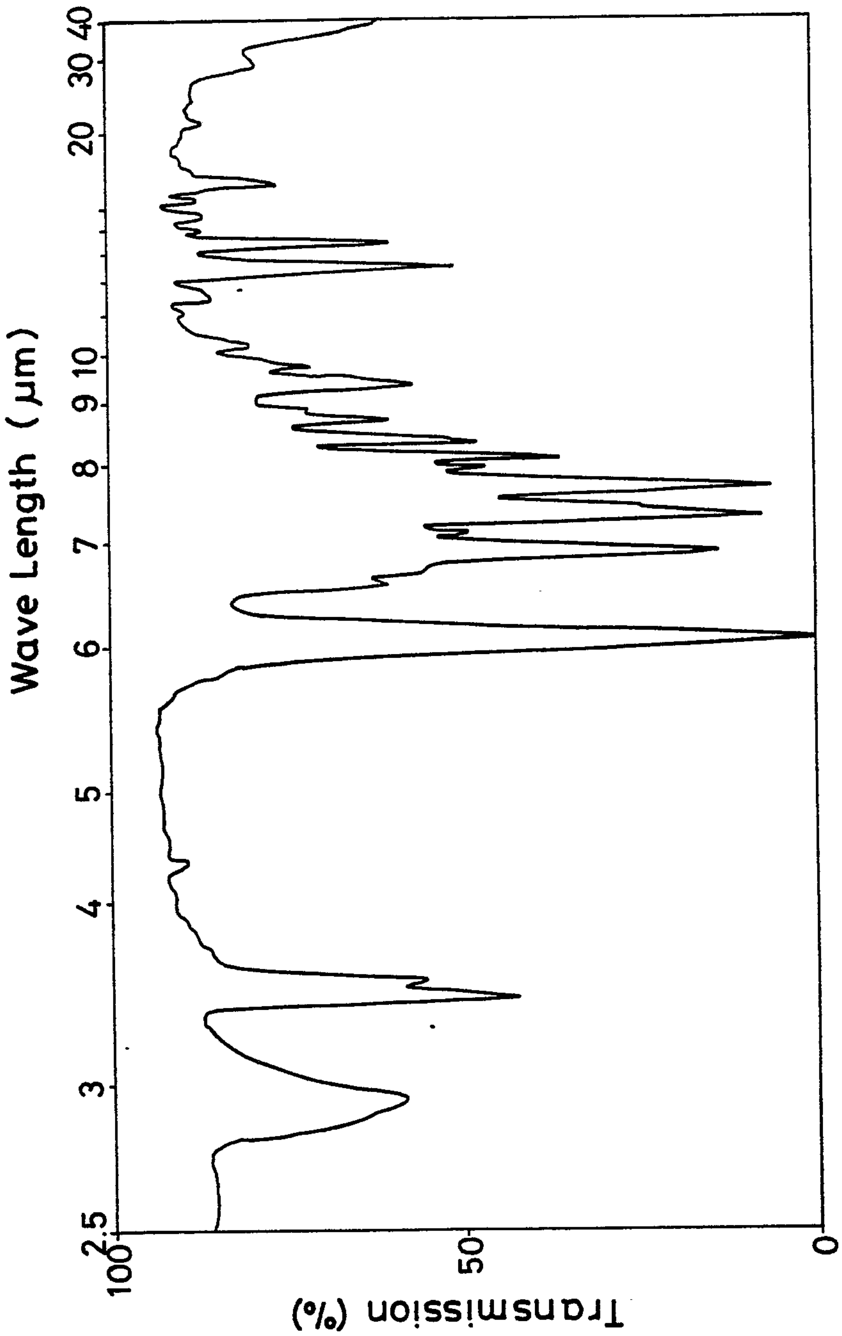

Fig. 5

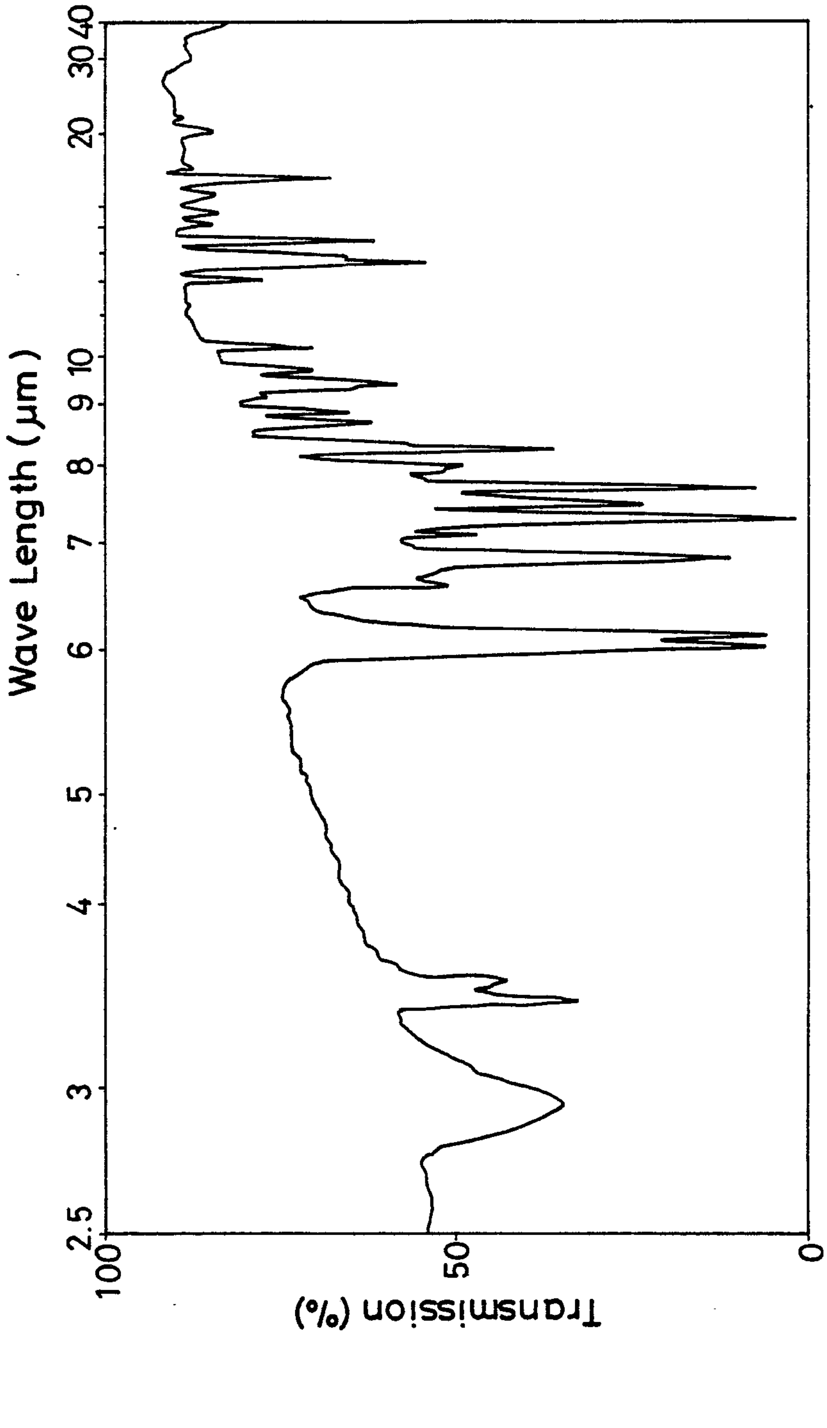

Fig. 6